# EUROPEAN PATENT SPECIFICATION

(11) **EP 1 646 364 B1**
(45) Date of publication and mention of the grant of the patent: **01.10.2008**
(21) Application number: 04740683.0
(22) Date of filing: 06.07.2004
(51) Int. Cl.: A61K 9/12, A61K 31/46

(54) **HFC SOLUTION FORMULATIONS CONTAINING AN ANTICHOLINERGIC**
HFC LÖSUNGSFORMULIERUNGEN ENTHALTEND EIN ANTICHOLINERGIKUM
PREPARATIONS DE HFC EN SOLUTION CONTENANT UN ANTICHOLINERGIQUE

(30) Priority: 11.07.2003 EP 03015834
(43) Date of publication of application: 19.04.2006
(60) Divisional application: 08104162.6
(73) Proprietor: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: SCHMIDT, Friedrich, 55218 Ingelheim (DE)
(74) Representative: Hammann, Heinz
(86) International application number: PCT/EP2004/007355
(87) International publication number: WO 2005/004844

(56) References cited:
- WO-A-02/32899
- WO-A-94/13262
- WO-A-03/057694
- WO-A-20/04054580

## Description

This invention relates to stable pharmaceutical solution formulations suitable for aerosol administration containing an anticholinergic of formula **1** 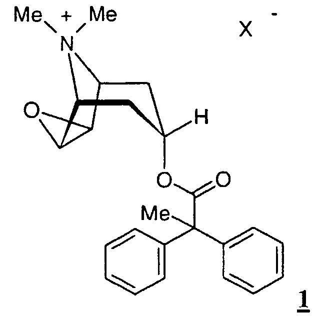 wherein X⁻ is a pharmaceutically acceptable anion. More particularly, this invention relates to stable pharmaceutical solution formulations suitable for aerosol administration containing an anticholinergic of formula **1** in an environmentally safe hydrofluorocarbon (HFC) as a propellant, together with an organic compound as a cosolvent wherein either an inorganic acid or an organic acid is added to the aerosol solution formulation.

### BACKGROUND OF THE INVENTION

The compounds of formula **1** are known from WO 02/32899. They represent highly effective anticholinergics with a long-lasting activity which can be used to treat respiratory complaints, particularly COPD (chronic obstructive pulmonary disease) and asthma.

For treating the abovementioned complaints, it is useful to administer the active substance by inhalation. In addition to the administration of broncholytically active compounds in the form of inhalable powders containing the active substance the administration of active substances can also occur in form of hydrofluorocarbon containing aerosol solution formulations.

The administration of aerosol formulations of medicaments by means of pressurized, metered-dose inhalers (MDIs) is used widely in therapy, such as in the treatment of obstructive airway diseases and asthma. Compared with oral administration, inhalation provides more rapid onset of action while minimizing systemic side effects. Aerosol formulations can be administered by inhalation through the mouth or topically by application to the nasal mucosa.

Formulations for aerosol administration via MDIs can be solutions or suspensions. Solution formulations offer the advantage of being homogeneous in nature with the medicament and excipient completely dissolved in the propellant vehicle. Solution formulations also obviate physical stability problems associated with suspension formulations and thus assure more consistent uniform dosage administration while also eliminating the need for surfactants.

The administration of aerosol solution formulations via MDIs is dependent upon the propulsive force of the propellant system used in its manufacture. Traditionally, the propellant comprised a mixture of chlorofluorocarbons (CFCs) to provide the desired solubility, vapor pressure, and stability of the formulation. However, since it has been established in recent years that CFCs are environmentally harmful because they contribute to the depletion of the Earth's ozone layer, it is desirable to substitute environmentally safe hydrofluorocarbon (HFC) propellants or other non-chlorinated propellants for environmentally harmful CFC propellants in aerosol inhalation formulations. For example, U.S. Patent No. 4,174,295 discloses the use of propellant systems consisting of combinations of HFCs, which may also contain a saturated hydrocarbon component, suitable for application in the fields of home products such as hair lacquers, anti-perspiration products, perfumes, deodorants, paints, insecticides and the like.

It is known in the art that certain HFCs have properties suitable for use as propellants for the aerosol administration of medicaments. For example, published European patent Application No. 0 372 777 (EPO89312270.5) describes the use of 1,1,1,2-tetrafluoroethane (HFC-134(a)) in combination with at least one "adjuvant" (a compound having a higher polarity than the HFC-134(a)) and a surface active agent to prepare suspension and solution formulations of medicaments suitable for administration by the aerosol route. Also, PCT Published Application No. W091/11496 (PCT/EP91/00178) discloses the use of 1,1,1,2,3,3,3-heptafluoropropane (HFC-227), optionally mixed with other propellant components, for use in preparing suspension aerosol formulations of medicaments. US-A-2 868 641 and US-A-3 282 781 disclose aerosol compositions comprising a medicament (epinephrine or isoproterenol HCl), a cosolvent, a propellant and ascorbic acid as anti-oxidant. European Patent EP 673 240 B1 proposes the addition of acids to medicinal aerosol formulations in order to provide for the stabilization of the medicament.

### DESCRIPTION OF THE INVENTION

The term "aerosol solution formulation" means a pharmaceutical formulation of a medicament suitable for aerosol administration wherein the medicament and excipients are completely dissolved.

The term "stabilized aerosol solution formulation" means an aerosol solution formulation which exhibits substantial chemical stability over time.

The present invention provides stabilized aerosol solution formulations comprising a salt of formula **1** 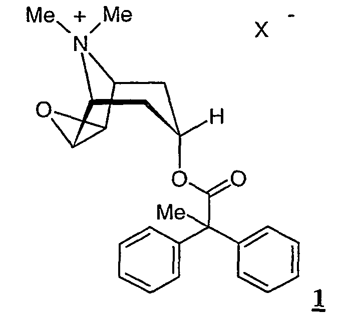 wherein
- X -: denotes an anion, preferably an anion selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate,
an HFC propellant, a cosolvent, and an inorganic or an organic acid, characterized in that the concentration of the acid is in a range that corresponds with a pH range of 2.5 - 5.5 in aqueous solution.

In a preferred embodiment of the invention the aerosol solution formulations contain those salts of formula **1** wherein
- X -: denotes an anion with a single negative charge selected from among the chloride, bromide, 4-toluenesulphonate and methanesulphonate, preferably bromide.

In another preferred embodiment of the invention the the aerosol solution formulations contain those salts of formula **1** wherein
- X -: denotes an anion with a single negative charge selected from among the chloride, bromide and methanesulphonate, preferably bromide.

In a yet another preferred embodiment of the invention the aerosol solution formulations contain the salt of formula **1** wherein X - denotes bromide.

The salts **1** may possibly be present in the form of their solvates or hydrates.
In preferred aerosol solution formulations according to the invention the concentration of the acid is in a range that corresponds with a pH range of 3.0 - 5.0, more preferred 3.5 - 4.5 in aqueous solution.

A small amount of water (up to about 5%, preferably up to about 3 % by weight, ) may also be present in the propellant/cosolvent system.

Particular preferred the aerosol solution formulations according to the invention contain one or more, preferably one salt of formula **1** as the single active ingredient.

Within the scope of the present invention, any reference to the compound **1'** is to be regarded as a reference to the pharmacologically active cation of the following formula **1'** contained in the salts 1 :

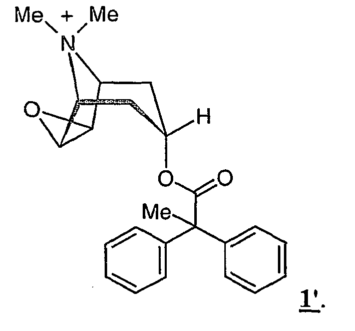

The aerosol solution formulation according to the invention preferably contains 0.00008 to 4 %, preferably 0.0004 to 1,6 %, more preferably 0.0008 to 0.8 % of the pharmacologically active cation **1'.**

If the particularily preferred salt **1,** the bromide, is used, the aforementioned amounts of the cation **1** correspond to 0.000097 to 4,8 % **1** (in form of the bromide), preferably 0.00048 to 1,94 %, more preferably 0.00097 to 0.97 % **1** (in form of the bromide).

Suitable HFC propellants are those which, when mixed with the cosolvent(s), form a homogeneous propellant system in which a therapeutically effective amount of the medicament can be dissolved. The HFC propellant must be toxicologically safe and must have a vapor pressure which is suitable to enable the medicament to be administered via a pressurized MDI. Additionally, the HFC propellant must be compatible with the components of the MDI device (such as containers, valves, and sealing gaskets, etc.) which is employed to administer the medicament. Preferred HFC propellants are 1,1,1,2-tetrafluoroethane (HFC-134(a)) and 1,1,1,2,3,3,3,-heptafluoropropane (HFC-227). HFC-134(a) is particularly preferred. Other examples of HFC propellants are HFC-32 (difluoromethane), HFC-143(a) (1,1,1-trifluoroethane), HFC-134 (1,1,2,2-tetrafluoroethane), and HFC-152a (1,1-difluoroethane). It will be apparent to those skilled in the art that non-halogenated hydrocarbon propellants may be used in place of the HFC propellants in the present invention. Examples of non-halogenated hydrocarbons are saturated hydrocarbons, including propane, n-butane, and isobutane, and ethers, including diethyl ether. It will also be apparent to those skilled in the art that, although the use of a single HFC propellant is preferred, a mixture of two or more HFC propellants, or a mixture of at least one HFC propellant and one or more non-CFC propellants, may be employed in the aerosol solution formulation of the present invention.

The acid in the formulations according to the invention may be any inorganic or mineral acid, for example, hydrochloric acid, sulfuric acid, nitric acid, or phosphoric acid, or the like. From the aforementioned acids hydrochloric acid and phosphoric acid are of particular interest. The acid may also be selected from the group of acids known to those skilled in the art as organic acids, which are in most cases considered to be weak acids relative to the inorganic acids. Representative of this group and preferred in this invention are ascorbic acid, citric acid, lactic acid, malic acid, benzoic acid and tartaric acid. According to this invention, citric acid and ascorbic acid are the most preferred organic acids.

The formulations according to the invention can be prepared in analogy to methods known in the art.

If desired, pharmaceutically acceptable excipients can be included in the aerosol solution formulations of the present invention. For example, a soluble surface active agent can be added in order to improve the performance of valve systems employed in the MDI devices used for the aerosol administration of the formulations. Examples of preferred surface active agents are sorbitan trioleate, lecithin, isopropylmyristate, ethoxylated glycerol trioleates and alkyl polyglycosides. Other suitable lubricants are well known in the art (see, for example, Published European Patent Application No. 0372777 (EPO 893122705)). Other excipients are: (a) antioxidants, for example ascorbic acid and tocopherol; (b) taste masking agents, for example, menthol, sweeteners, and artificial or natural flavors; and (c) pressure modifying agents, for example, n-pentane, iso-pentane, neo-pentane, and n-hexane.

Examples of cosolvents applicable within the formulations according to the invention are: alcohols, for example, ethyl alcohol, isopropyl alcohol, and benzyl alcohol; glycols for example, propylene glycol, polyethylene glycols, polypropylene glycols, glycol ethers, and block copolymers of oxyethylene and oxypropylene; and other substances, for example, glycerol, polyoxyethylene alcohols, polyoxtethylene fatty acid esters, and glycofurols (for example glycofurol 75).
Examples of cosolvents that may be inert to interaction with the medicament(s) are hydrocarbons, for example, n-propane, n-butane, isobutane, n-pentane, iso-pentane, neo-pentane, and n-hexane; and ethers, for example, diethyl ether.
A preferred cosolvent according to this invention is ethyl alcohol (ethanol).

The amount of cosolvent is preferably in the range of 5 - 50% (w/w) of the total composition. More preferably, the amount of co-solvent in the formulation according to the invention is in the range of 10 - 40 % (w/w), preferably in the range of 15 - 30 %.

As mentioned hereinbefore the formulations according to the invention may contain water. One preferred embodiment of the invention pertains to formulations that contain water in an amount of up to 5% (w/w), preferably of up to 3 % (w/w). Another preferred embodiment of the invention is directed to formulations that do not contain any water. In these water-free formulations the amount of cosolvent is preferably in the range of about 20 - 50% (w/w), more preferably in the range of about 30 - 40% (w/w).

The formulations according to the invention can be administered with inhalers known in the art (Metered dose inhalers = MDIs).

In another aspect the invention is directed to the use of an aerosol solution formulation as described hereinbefore for the manufacture of a medicament for the treatment of respiratory complaints, particularly COPD (chronic obstructive pulmonary disease) and asthma.

In yet another aspect the invention is directed to a method for treatment of respiratory complaints, such as in particular COPD (chronic obstructive pulmonary disease) or asthma, characterized by the administration of an aerosol solution formulation as described hereinbefore.

The following Examples serve to illustrate the present invention further.

### I. Formulation examples

| **A)** | |
|---|---|
| **Component** | **Concentration [% w/w]** |
| **1** (wherein X⁻ is bromide) | 0.2 |
| Ethanol abs. (USP) | 25 |
| Water (purified, USP) | 1.0 |
| Citric acid (USP) | 0.003 |
| HFC-134a | 73.797 |

| B) | |
|---|---|
| **Component** | **Concentration [% w/w]** |
| **1** (wherein X⁻ is bromide) | 0.1 |
| Ethanol abs. (USP) | 20.0 |
| Aqueous HCl 0.01 mol/l (USP) | 2.0 |
| HFC-134a | 77.9 |

| **C)** | |
|---|---|
| **Component** | **Concentration [% w/w]** |
| **1** (wherein X⁻ is bromide) | 0.1 |
| Ethanol abs. (USP) | 15.0 |
| Water (purified, USP) | 2.0 |
| Citric acid (USP) | 0.004 |
| HFC-227 | 82.896 |

| **D)** | |
|---|---|
| **Component** | **Concentration [% w/w]** |
| **1** (wherein X⁻ is bromide) | 0.2 |
| Ethanol abs. (USP) | 30.0 |
| Water (purified, USP) | 1.0 |
| Ascorbic acid (USP) | 0.005 |
| HFC-134a | 68.795 |

| **E)** | |
|---|---|
| **Component** | **Concentration [% w/w]** |
| **1** (wherein X- is bromide) | 0.05 |
| Ethanol abs. (USP) | 40.0 |
| citric acid (USP) | 0.004 |
| HFC-227 | 59.991 |

The aforementioned formulations can be prepared by conventional methods known in the state of the art.

## Claims

1. Aerosol solution formulation comprising a salt of formula **1** 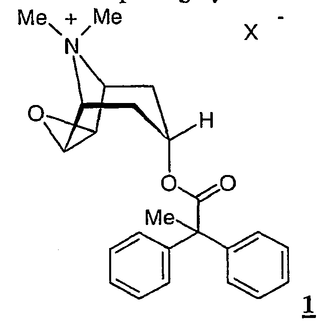 wherein
X - denotes an anion, preferably an anion selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate,
an HFC propellant, a cosolvent, and an inorganic or an organic acid, **characterized in that** the concentration of the acid is in a range that corresponds with a pH range of 2.5 - 5.5 in aqueous solution.

2. Aerosol solution formulation according to claim 1, **characterized in that** it contains 0.00008 to 4 % the pharmacologically active cation of formula **1'**

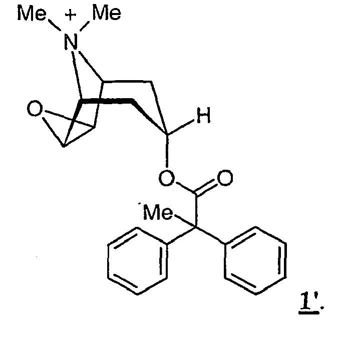

3. Aerosol solution formulation according to claim 1 or 2, **characterised in that** the HFC propellant is selected from HFC-134(a), HFC-227, HFC-32, HFC-143(a), HFC-134, HFC-152a and mixtures thereof.

4. Aerosol solution formulation according to claim 1, 2 or 3, **characterised in that** the acid is selected from the inorganic acids hydrochloric acid, sulfuric acid, nitric acid, and phosphoric acid.

5. Aerosol solution formulation according to claim 1, 2 or 3, **characterised in that** the acid is selected from the organic acids ascorbic acid, citric acid, lactic acid, malic acid, benzoic acid, and tartaric acid.

6. Aerosol solution formulation according to one of claims 1 to 5, **characterised in that** it contains water in an amount of up to 5%.

7. Aerosol solution formulation according to one of claims 1 to 6, **characterised in that** it contains as a cosolvent alcohols, glycols, glycol ethers, block copolymers of oxyethylene and oxypropylene, glycerol, polyoxyethylene alcohols, polyoxtethylene fatty acid esters or glycofurols.

8. Aerosol solution formulation according to one of claims 1 to 7, **characterised in that** the cosolvent is present in an amount in the range of 5 - 50% (w/w).

9. Aerosol solution formulation according to one of claims 1 to 5, 7 or 8, **characterised in that** it contains no water.

10. Use of an aerosol solution formulation according to one of claims 1 to 9 for the manufacture of a medicament for the treatment of respiratory complaints.

## Patentansprüche

1. Aerosol-Lösungsformulierung, umfassend ein Salz der Formel **1** 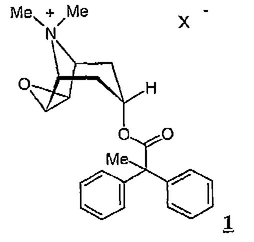 worin
X⁻ ein Anion darstellt, bevorzugt ein Anion, ausgewählt aus der Gruppe, bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat,
ein HFC-Treibmittel, ein Co-Lösungsmittel und eine anorganische oder organische Säure, **dadurch gekennzeichnet, dass** die Konzentration der Säure in einem Bereich liegt, der einem pH-Bereich von 2,5-5,5 in einer wässerigen Lösung entspricht.

2. Aerosol-Lösungsformulierung nach Anspruch 1, **dadurch gekennzeichnet, dass** sie 0,00008 bis 4% des pharmakologisch wirksamen Kations der Formel **1'** enthält:

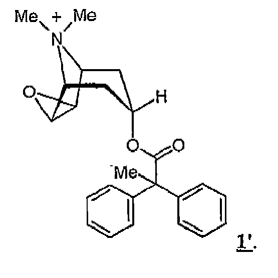

3. Aerosol-Lösungsformulierung nach Anspruch 1 oder 2, **dadurch gekennzeichnet, dass** das HFC-Treibmittel ausgewählt ist aus HFC-134(a), HFC-227, HFC-32, HFC-143(a), HFC-134, HFC-152a und Mischungen hiervon.

4. Aerosol-Lösungsformulierung nach Anspruch 1, 2 oder 3, **dadurch gekennzeichnet, dass** die Säure ausgewählt ist aus den anorganischen Säuren Salzsäure, Schwefelsäure, Salpetersäure und Phosphorsäure.

5. Aerosol-Lösungsformulierung nach Anspruch 1, 2 oder 3, **dadurch gekennzeichnet, dass** die Säure ausgewählt ist aus den organischen Säuren Ascorbinsäure, Zitronensäure, Milchsäure, Äpfelsäure, Benzoesäure und Weinsäure.

6. Aerosol-Lösungsformulierung nach einem der Ansprüche 1 bis 5, **dadurch gekennzeichnet, dass** sie Wasser in einer Menge von bis zu 5% enthält.

7. Aerosol-Lösungsformulierung nach einem der Ansprüche 1 bis 6, **dadurch gekennzeichnet, dass** sie als ein Co-Lösungsmittel Alkohole, Glykole, Glycolether, Blockcopolymere von Oxyethylen und Oxypropylen, Glycerin, Polyoxyethylenalkohole, Polyoxyethylenfettsäureester oder Glycofurole enthält.

8. Aerosol-Lösungsformulierung nach einem der Ansprüche 1 bis 7, **dadurch gekennzeichnet, dass** das Co-Lösungsmittel in einer Menge im Bereich von 5-50% (Gew./Gew.) enthalten ist.

9. Aerosol-Lösungsformulierung nach einem der Ansprüche 1 bis 5, 7 oder 8, **dadurch gekennzeichnet, dass** sie kein Wasser enthält.

10. Verwendung einer Aerosol-Lösungsformulierung nach einem der Ansprüche 1 bis 9 zur Herstellung eines Arzneimittels zur Behandlung von respiratorischen Beschwerden.

## Revendications

1. Formulation de solution d'aérosol comprenant un sel de formule **1** 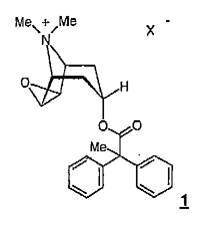 où
X⁻ désigne un anion, de préférence un anion choisi dans le groupe consistant en chlorure, bromure, iodure, sulfate, phosphate, méthanesulfonate, nitrate, maléate, acétate, citrate, fumarate, tartrate, oxalate, succinate, benzoate et p-toluènesulfonate,
et un propulseur HFC, un cosolvant, et un acide inorganique ou organique, **caractérisée en ce que** la concentration de l'acide est dans une plage qui correspond à une plage de pH de 2,5 - 5,5 en solution aqueuse.

2. Formulation de solution d'aérosol selon la revendication 1 **caractérisée en ce qu'**elle contient 0,00008 à 4 % de cation pharmacologiquement actif de formule **1'**

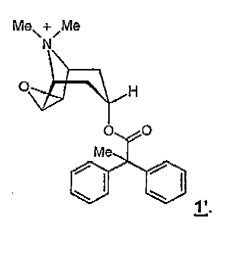

3. Formulation de solution d'aérosol selon la revendication 1 ou 2 **caractérisée en ce que** le propulseur HFC est choisi parmi HFC-134(a), HFC-227, HFC-32, HFC-143(a), HFC-134, HFC-152a et leurs mélanges.

4. Formulation de solution d'aérosol selon la revendication 1, 2 ou 3 **caractérisée en ce que** l'acide est choisi parmi les acides inorganiques acide chlorhydrique, acide sulfurique, acide nitrique et acide phosphorique.

5. Formulation de solution d'aérosol selon la revendication 1, 2 ou 3 **caractérisée en ce que** l'acide est choisi parmi les acides organiques acide ascorbique, acide citrique, acide lactique, acide malique, acide benzoïque et acide tartrique.

6. Formulation de solution d'aérosol selon l'une des revendications 1 à 5 **caractérisée en ce qu'**elle contient de l'eau en une quantité pouvant atteindre 5 %.

7. Formulation de solution d'aérosol selon l'une des revendications 1 à 6 **caractérisée en ce qu'**elle contient comme cosolvant des alcools, des glycols, des éthers de glycol, des copolymères séquencés d'oxyéthylène et d'oxypropylène, du glycérol, des alcools polyoxyéthylénés, des esters d'acides gras polyoxyéthylénés ou des glycofurols.

8. Formulation de solution d'aérosol selon l'une des revendications 1 à 7 **caractérisée en ce que** le cosolvant est présent en une quantité dans la plage de 5 - 50 % (m/m).

9. Formulation de solution d'aérosol selon l'une des revendications 1 à 5,7 ou 8 **caractérisée en ce qu'**elle ne contient pas d'eau.

10. Utilisation d'une formulation de solution d'aérosol selon l'une des revendications 1 à 9 pour la fabrication d'un médicament pour le traitement des affections respiratoires.
